(19) Europäisches Patentamt
European Patent Office
Office européen des brevets

(11) EP 1 280 766 B1

(12) EUROPÄISCHE PATENTSCHRIFT

(45) Veröffentlichungstag und Bekanntmachung des Hinweises auf die Patenterteilung:
**22.12.2004 Patentblatt 2004/52**

(21) Anmeldenummer: **01931186.9**

(22) Anmeldetag: **08.05.2001**

(51) Int Cl.⁷: **C07C 279/12**, C07C 277/08, C08G 73/00, A01N 47/44, A01N 61/00

(86) Internationale Anmeldenummer:
**PCT/AT2001/000134**

(87) Internationale Veröffentlichungsnummer:
**WO 2001/085676 (15.11.2001 Gazette 2001/46)**

# (54) BIOZIDE POLYMERE AUF DER BASIS VON GUANIDIN-SALZEN

BIOCIDAL POLYMERS BASED ON GUANIDINE SALTS

POLYMERES BIOCIDES A BASE DE SELS DE GUANIDINE

(84) Benannte Vertragsstaaten:
**AT BE CH CY DE DK ES FI FR GB GR IE IT LI LU MC NL PT SE TR**
Benannte Erstreckungsstaaten:
**AL LT LV MK RO SI**

(30) Priorität: **11.05.2000 AT 8262000**
**23.10.2000 AT 18182000**

(43) Veröffentlichungstag der Anmeldung:
**05.02.2003 Patentblatt 2003/06**

(73) Patentinhaber: **POC Polymer Produktions GmbH 1030 Wien (AT)**

(72) Erfinder:
- **SCHMIDT, Oskar, J.**
  **A-1010 Wien (AT)**
- **SCHMIDT, Andreas**
  **CH-4153 Reinach (CH)**
- **TOPTCHIEV, Dimitri**
  **Moskau B-71, 117071 (RU)**

(74) Vertreter: **Rippel, Andreas, Dipl.-Ing. et al**
**Patentanwalt Dipl.-Ing. Rippel**
**Kommandit-Partnerschaft**
**Maxingstrasse 34**
**1130 Wien (AT)**

(56) Entgegenhaltungen:
**EP-A- 0 439 698** **EP-A- 0 439 699**
**AT-B- 406 163**

- **DATABASE WPI Section Ch, Week 199614 Derwent Publications Ltd., London, GB; Class A26, AN 1996-138041 XP002174283 & RU 2 039 735 C (GEMBITSKII P A), 20. Juli 1995 (1995-07-20)**
- **DATABASE WPI Section Ch, Week 199430 Derwent Publications Ltd., London, GB; Class A26, AN 1994-247737 XP002174284 & SU 1 816 769 A (MOSVODKANALNIIPROEKT RES DES INST), 23. Mai 1993 (1993-05-23)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 280 766 B1

## Beschreibung

Die Erfindung bezieht sich auf biozide Polymere auf der Basis von Guanidin-Salzen, welche als Desinfektionsmittel in der Medizin, Veterinärmedizin, bei der Abwasserreinigung, im Haushalt sowie in allen Branchen der Wirtschaft, in denen biozide Präparate benötigt werden, Anwendung finden. Weiters betrifft die Erfindung ein Verfahren zur Gewinnung von solchen Salzen.

Zweck vorliegender Erfindung ist, ein hochmolekulares, homogenes und reines Desinfektionsmittel auf Guanidinsaiz-Basis zu gewinnen, welches über eine geringe Toxizität, jedoch eine hohe biozide Aktivität, eine größere relative Molekularmasse, eine höhere Hydrophylie sowie die Eigenschaften oberflachenaktiver Substanzen aufweist.

Erreicht wird dies dadurch, daß die Guanidinderivate ein Produkt der Polykondensation eines Guanidin-Säureadditionssalzes mit Diaminen, welche Polyoxyalkylenketten zwischen zwei Aminogruppen enthalten, darstellen.

Die Gewinnung dieses Produktes erfolgt erfindungsgemäß, dadurch, daß die flüssigen Diamine der Polykondensation mit Guanidin-Salzen im Medium von flüssigen Diamin unter ständiger Durchmischung von Begin bis zum Abschluß der Reaktion bei erhöhter Temperatur und mit einer Dauer der Polykondecuation von 9-16 Stunden, je nach Art des verwendeten Diamins, unterworfen werden.

Bei der vorgeschlagenen Erfindung wird im Bereich der Synthese die Durchführung der Reaktion in flüssiger Phase bei ständiger Durchmischung von Beginn bis zum Abschluß gewährleistet. Dadurch wird die erforderliche Homogenisierung des Systems, die Einhaltung . des erforderlichen Molverhältnisses der Reagenzien im Verlauf der Reaktion, d.h. eine hohe Produktqualität, sowie die Vereinfachung der gerätetechnischen Ausstattung des Prozesse und eine Erleichterung der Sicherheitsanforderungen, die an den Prozeß gestellt werden, ermöglicht.

Insbesondere werden zum Beispiel Triethylenglykoldiamin mit einer relativen Molekülmasse von 148 sowie Polyoxyalkylendiamine verschiedener Zusammensetzung mit den Guanidin-Säureadditionssalzen umgesetzt. Es kann dabei eine umfangreiche Reihe biozider Polymerer von polykationischer Natur gewonnen werden, welche eine hohe Aktivität und eine geringe Toxizität aufweisen. Diese Polymere können in der Praxis als Desinfektionsmittel auf verschiedenen Gebieten verwendet werden.

Beispiele von für die Durchführung der Polykondensationsreaktion mit dem Triethylenglykoldiamin und den Polyoxyalkylendiaminen geeigneten Guanidin-Säureadditionssalzen sind Salze anorganischer und organischer Säuren, wie beispielsweise das Hydrochlorid, Dihydrogenphosphat, Carbonat, Sorbat, Nitrat, Hydroacetat, Glukonat, Citrat und Silikat.

Die vorgeschlagenen Diamine sind Flüssigkeiten mit einem hohen Grad an Hydrophilie, einer geringen Flüchtigkeit und einem relativ niedrigen Dampfdruck, was gewährleistet, daß im Reaktionsverlauf keine Diamindämpfe weder im Reaktionsapparat, noch in der Umwelt vorhanden sind und es im Reaktionsverlauf nicht zu einer Veränderung des Molverhältnisses der Reagenzien führt. Die Verwendung der angeführten Diamine ermöglicht die Durchmischung im flüssigen Reaktionsmedium von Anfang bis Abschluß der Reaktion, d.h. eine effektive Kontrolle des Prozesses.

Die Reaktion erfolgt vorzugsweise bei einem Ausgangsmolverhältniss der Reagenzien Guanidinsalz und Diamin (DA) von 1 : 1 bei Temperaturen von 140 °C — 190 °C je nach Art des verwendeten Diamins unter ständiger Durchmischung. Für die Reaktion wird ein handelsübliches reines (99 % Reinheit) Guanidinsalz verwendet. Als Ergebnis wurden neue polymere Biozide auf Guanidinsalz-Basis — die wasserlöslichen Polyoxyalkylen-Guanidinsalze - gewonnen, die eine erhöhte Bakterizidität, sowie einen besseren Grad an Hydrophilie und ausgeprägte Eigenschaften polymerer oberflächenaktiver Substanzen aufweisen. Der Wert der Mindesthemmkonzentration in % für E.coli beträgt 0,00008, d.h. er ist wesentlich besser als bei Polyhexamethylenguanidin (PHMG), die relative Molekularmasse Mw = 13.500 ist höher als bei PHMG, die Toxizität ist geringer, nämlich $LD_{50}$= 3400 mg/kg für Ratten, im Gegensatz zu PHMG mit $LD_{50}$ = 2500 mg/kg.

Die erzielten Ergebnisse können an den folgenden Beispielen demonstriert werden, wobei den Beispielen 1 bis 4 die angeschlossene Tabelle zugeordnet ist:

<u>Beispiel 1:</u>

In einen mit einem Mischwerk und einem Luftkühler ausgestatteten Dreihalskolben mit einem Fassungsvermögen von 250 ml werden bei einer Raumtemperatur von 20° C 25,28 (0,17 Mol) von flüssigem Diamin - Triethylenglykoldiamin (TEDA) ($H_2N$ -$CH_2$ $CH_2$- O - $CH_2CH_2$ - O - $CH_2CH_2$ - $NH_2$) eingefüllt. Anschließend werden 16,34 g (0,17 Mol) pulverförmiges Guanidin-Hydrochlorid (GHCI) in den Kolben zugegeben. Unter ständiger Durchmischung wird der Kolben 5 Stunden lang bei einer Temperatur von 150 C erhitzt. Die Viskosität des Reaktionsmediums steigt je nach dem Fortgang der Reaktion, die mit der Abgabe von gasförmigem Ammoniak einhergeht. In diesem Stadium des Prozesses wurde eine Probe aus dem Reaktionsapparat entnommen. Dieses harzförmige, gelbliche Produkt, ist hydrophil und löst sich rasch zur Gänze in Wasser auf. Die Einwaage eines getrockneten Musters dieses Produktes (Muster 1a) wurde in Wasser aufgelöst und ihre charakteristische Viskosität wurde im Ubbelohde-Viskosimeter gemessen. Diese beträgt [η] = 0,04 dl/g, was unter Beweis stellt, daß bereits in diesem Reaktionsstadium ein polymeres

Produkt mit einem Gewichtsmittel der relativen Molekülmasse Mw = 2500 gebildet wird. Dieses Produkt weist bereits biozide Eigenschaften auf (s. Tabelle).

Im weiteren wird die Erhitzung bei der Durchmischung des flüssigen Reaktionsgemisches bei einer Temperatur von 170 °C innerhalb von 9 Stunden fortgesetzt. Dabei gehen auch die Gasentwicklung und die Erhöhung der Viskosität des Reaktionsmediums weiter. Nach den angeführten 9 Stunden Reaktionszeit bei einer Temperatur von 170 °C wurde dem Reaktionssystem noch eine andere Probe Nr. 1 b) entnommen. Die Farbintensität der polymeren Probe erhöhte sich auf hellbraun. Aus der Messung der charakteristischen Viskosität dieses Musters 1 b) ergab sich der Wert [η] = 0,07 dl/g, was einem Gewichtsmittel der relativen Molekülmasse Mw = 5800 entspricht und was bedeutet, daß die relative Molekülmasse im Verlauf der Reaktion ansteigt. Ebenso erhöht sich die Bakterizidität, s. Tabelle. Da nach einer Exposition von 9 Stunden bei einer Temperatur von 170 °C noch eine Gasentwicklung zu beobachten war, d.h. die Reaktion noch nicht abgeschlossen war, wurde die Erhitzung des Reaktionssystems bei dieser Temperatur von 170 °C noch 4 Stunden zusätzlich fortgesetzt. Danach kamen die Gasentwicklung und die Reaktion zum Abschluß.

Anschließend wurde noch ein Polymermuster 1 c) entnommen und es wurden dessen charakteristische Viskositätswerte gemessen, [η] = 0,085 dl/g, was einer relativen Molekülmasse von Mw = 9100 entspricht. Das Reaktionsprodukt löst sich rasch zur Gänze in Wasser auf und weist eine beträchtliche Hydrophilie auf.

Die Bestimmung der Bestandteile der einzelnen Elemente des polymeren Produkts hat folgende Ergebnisse gezeigt:

| | |
|---|---|
| es wurden ermittelt, in % | C-40,4; 40,25; N-19,7; 19,85; H - 7,4; 7,456. |

von flüssigem Polyoxypropylendiamin mit folgender Struktur eingefullt:

| | |
|---|---|
| Für $C_7N_3O_2Cl\ H_{16}$ wurde berechnet, in % | C-40, 1; N-20,04; H-7,63. |

Somit wurde ein neues polymeres Produkt gewonnen, welches in seiner Zusammensetzung dem Polytriethylenglykol-Cuanidin-Hydrochlorid entspricht. Das Endprodukt des beschriebenen Experimentes wurde mit einer quantitativen Ausbeute von 98, 7 % gewonnen. Es ist gering toxisch, orale Dosis bei Ratten $LD_{50}$ = 3100mg/kg, d.h. es weist eine wesentlich geringere Toxizität als PHMG (s. Tabelle) sowie eine hohe bakterizide Aktivität auf.

<u>Beispiel 2:</u>

Dieselben Ausgangsreagenzien in derselben Menge und in demselben Molverhältnis wie in Beispiel 1 werden in den Reaktionskolben eingebracht. Die Reaktion erfolgte unter ständiger Durchmischung von Anfang bis zum Abschluß bei einer Temperatur von 150 °C im Laufe von 25 Stunden solange, bis kein Ammoniak mehr abgegeben wird. Das erhaltene Reaktionsprodukt ist wasserlöslich, von hellbrauner Farbe mit einer Ausbeute von 99,1 %. In der Zusammensetzung seiner Elemente entspricht das Reaktionsprodukt dem Polyethylenglykol-Guanidin-Hydrochlorid.

Zusammensetzung der Elemente:

| | |
|---|---|
| es wurden ermittelt, in % | C - 40,7; N - 19,65; H - 7,6 |
| in % wurde berechnet | C - 4030 1; N - 20,04; H - 7,63 |

Die charakteristische Viskosität dieses Musters (Muster 2) wurde gemessen mit [η] = 0,11 (Mw ~ 11800). Dieses Muster weist eine höhere bakterizide Aktivität sowie eine geringere Toxizität als PHMG auf (s. Tabelle).

<u>Beispiel 3:</u>

In einen mit einem Mischwerk und einem Luftkühler ausgestatteten Dreihalskolben mit einem Fassungsvermögen vn 250 ml werden bei einer Raumtemperatur von 20 °C 48 g (0,208 Mol)

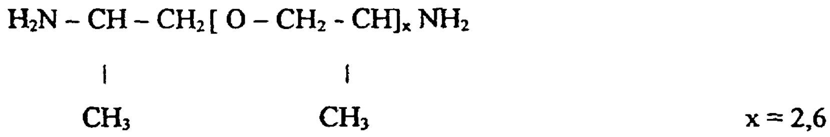

x ≈ 2,6

mit einem Molekulargewciht von 230 und einer äquimolekularen Menge von pulverförmigem Guanidin-Hydrochlorid (GHCl) von 19 g (0,208 Mol). Das Gemisch wird unter ständiger Durchmischung erhitzt, zunächst 2 Stunden lang bei einer Temperatur von 150 °C und anschließend 9 Stunden lang bei einer Temperatur von 170 °C. In diesem Stadium wurde aus dem Reaktionsgemisch das Muster 3 a) ein hydrophiles, klebriges Produkt von hellbrauner Farbe entnommen und dessen charakteristisch Viskosität gemessen: [η] = 0,045 dl/g, was einem Molekulargewicht $\overline{M}w \sim 3000$ entspricht. Im weiteren wurde die Erhitzung bei dieser Temperatur von 170 °C noch 9 Stunden lang fortgesetzt, bis kein Ammoniak mehr abgegeben wurde, d.h. bis zum Abschluß der Reaktion. Die Ausbeute des Endproduktes beträgt 98,9 %.

Gemäß den Angaben der Analyse der einzelnen Elemente entspricht das Endprodukt der geforderten Formel von Polyoxypropylen-Guanidin-Hydrochlorid.

| | |
|---|---|
| Ermittelt wurden in % | C - 50,85; N - 13,35; H - 9,6 |
| Berechnet wurden in % | C - 50,4; N - 13,57; H - 9,69. |

Es wurde die charakteristische Viskosität des Endproduktes der Reaktion, Muster 3 b) bestimmt, [η] = 0,12, was einem Molekulargewicht $\overline{M}w \sim 12500$ entspricht, d.h. höher als im Falle von PHMG.

Die Bestimmung der Bakterizidität (E.coli: Stamm Nr. 2590) für das Muster 3 b) hat im Vergleich zu PHMG dessen höhere biozide Aktivität sowie eine geringere Toxizität gezeigt (s. Tabelle).

<u>Beispiel 4:</u>

In einen mit einem Mischwerk und einem Luftkühler ausgestatteten Dreihalskolben mit einem Fassungsvermögen von 250 ml werden bei einer Raumtemperatur von 20 °C 124,8 g (0,208 Mol) eines flüssigen Diamins - Polyoxyethylendiamin/Polyoxypropilen - mit einem Molekulargewicht von 600 und folgender Strukturformel:

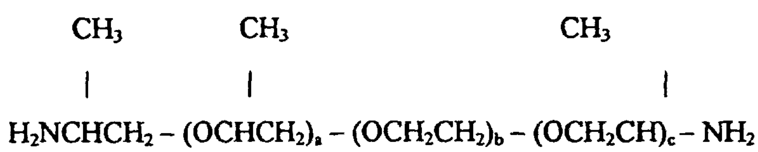

a+c=2,5, b=8,5

sowie 19 g (0,208 Mol) Guanidin-Hydrochlorid (GHCl) eingefüllt.

Weiters wurde bei ständiger Durchmischung das Reaktionsgemisch 25 Stunden lang bei einer Temperatur von 150 °C erhitzt. Im Verlauf der Reaktion kam es zu einer Abgabe von Ammoniak und einer Erhöhung der Viskosität des Reaktionssystems. Nach 25 Stunden Erhitzung wurde kein Ammoniak mehr abgegeben, d.h. die Reaktion kam zum Abschluß. Es wurde ein Reaktionsprodukt mit einer Ausbeute von 99,1 % abgegeben. Das ist ein Polymer von hellbrauner Farbe, das sich rasch und zur Gänze in Wasser auflöst. Für das gewonnene Polymer, das Muster 4, wurde die charakteristische Viskosität gemessen: [η] = 0,13, was einem Molekulargewicht $\overline{M}w \sim 13500$ entspricht, d.h. es wurde zum ersten Mal durch Polykondensat ein Polymer auf Basis von Guanidin-Hydrochlorid mit einer derartig hohen Molekulannasse gewonnen.

Laut den Angaben der Analyse der einzelnen Elemente entspricht das gewonnene polymere Produkt Polyoxyethylen-Guanidin-Hydrochlorid mit der oben angeführten Polyoxyethylen-Strukturgruppe.

| | |
|---|---|
| Ermittelt wurden in % | C - 53,1; H - 7,85; N - 6,95 |
| Berechnet wurden in % | C - 52,3; H - 7,87; N - 7,04 |

Das gewonnene Polymer weist eine geringere Toxizität sowie eine erhöhte Bakterizidität auf (s. Tabelle). Das

gewonnene Polymermuster verfugt ebenso über die ausgeprägte Eigenschaft eines oberflächenaktiven Polymers.

Es hat sich gezeigt, daß der für dieses Muster bestimmte Wert der Oberflächenspannung von 32 din/cm der Oberflächenspannung der bekannten oberflächenaktiven Substanz Dodezyl-Natriumsulfat nahekommt. (Hervorzuheben ist, daß bei den PHMG-Mustern keine ausgeprägten oberflächenaktiven Eigenschaften festgestellt werden konnten). Diese Eigenschaft einer oberflächenaktiven Substanz soll ein aktiveres Auftreten biozider Eigenschaften synthetisierter polymerer Produkte an der Phasentrennungsgrenze ermöglichen, und zwar bei der Behandlung (Desinfektion) von Oberflächen sowie bei deren Verwendung als Bestandteil von Waschmitteln.

<u>Beispiel 5:</u>

Gewinnung von Polytriethylenglykol-Guanidindihydrogenphosphat.

In einen Dreihalskolben mit einem Fassungsvermögen von 250 ml, versehen mit einem mechanischen Mischwerk und einem Luftkühler, werden bei Zimmertemperatur 47,5 g (0,32 Mol) flüssiges Triethylenglykoldiamin (relative Molekülmasse 148) eingebracht. Anschließend werden in den Kolben 50,24 g (0,32 Mol) pulverförmiges Guanidindihydrogenphosphat (relative Molekülmasse 157), d.h. im Molverhältnis der Reagenzien 1 : 1, zugesetzt. Der Kolben mit der flüssigen Reaktionsmasse wurde in ein Ölbad mit Temperaturregler getaucht. Die Reaktionsmasse wurde bei ständiger Durchmischung bei einer Temperatur von 170 °C innerhalb von 11 Stunden erhitzt. Von den ersten Minuten der angeführten Thermostatisierung an kam es zu einer intensiven Ammoniakabgabe (Färbung des Indikatorpapiers), was den Ablauf der Reaktion der Polykondensation beweist. Im Verlauf der Reaktion wird die Reaktionsmasse dickflüssig, visuell ist dabei Schaumbildung zu beobachten. Das Reaktionsgemisch verwandelt sich nach und nach in ein Harz von weißer Farbe, dessen Volumen das Volumen des flüssigen Ausgangsgemisches übersteigt. Sobald die Gasausscheidung des Ammoniaks beendet dies auch die Beendigung der Reaktion. Nach Abkühlung des Kolbens wird das Polymerharz mit Hilfe eines Spatels aus dem Kolben entfernt und in einem Mörser zu Pulver zerkleinert, welches eine höhere Hydrophobie im Vergleich zu Polyoxytriethylenglykol-Guanidinhydrochlorid aufweist, aber sich gleichzeitig dabei rasch in Wasser auflöst. Als Ergebnis des Versuchs wurden ca. 84,5 g des polymeren Endproduktes gewonnen. Es wurde die charakteristische Viskosität des gewonnenen Polymers gemessen: 0,4 N in einer wäßrigen Natriumchlorid-Lösung bei 25 °C. Die Viskosität beträgt [η] = 0,056 dl/g.

Elementaranalyse des gewonnenen Polymers:

Berechnet für $C_7N_3O_6PH_{18}$: C - 30,99 %, N - 15,49 %, O - 35,42 %, P - 11,44 %, H - 6,64 % Gefunden: C - 31,38 %, N - 15,25 %, P - 11,67 %, H - 6,49 %.
Die Analyse beweist die Übereinstimmung des gewonnenen Polymers mit der geforderten Struktur.
Es wurden die bioziden Eigenschaften des gewonnenen Polymers untersucht. Es wurde der Wert der Mindesthemmkonzentration in der Einheit (µg/ml) für zwei Bakterientypen bestimmt.
Im Falle von E-Coli-Bakterien beträgt dieser Wert 2,1 µg/ml. Im Falle von Ps aeruginosa - 6,2 µg/ml. Dies bestätigt den hohen Grad der bioziden Aktivität des gewonnenen Polymers. Es wurden auch einige toxikologischen Kennzahlen des Polymers untersucht, es wurde der Wert $LD_{50}$ (orale Dosis) für Ratten bestimmt. Dieser Wert beträgt 3200 mg/kg, was die geringe Toxizität des Polymers unter Beweis stellt.

<u>Beispiel 6:</u>

Gewinnung von Polytriethylenglykol-Guanidincarbonat.

In einen Dreihalskolben mit einem Fassungsvermögen von 1 1, versehen mit einem mechanischen Mischwerk und einem Luftkühler, werden bei Zimmertemperatur 148 g (1 Mol) flüssiges Triethylenglykoldiamin und anschließend 121 g (1 Mol) pulverförmiges Guanidincarbonat eingebracht. Der Kolben wird in einem Ölbad bei ständigem Durchmischen erhitzt, dabei erfolgt eine ziemlich homogene Vermischung der Reagenzien. Bei einer Temperatur von 140 °C setzt eine intensive Reaktion mit Ammoniakabgabe ein. Das Ausgangsgemisch wird bei dieser Temperatur (140 °C) während 15 Stunden gehalten. Anschließend wird es dickflüssig und verwandelt sich in eine hellgelbe schaumformige Masse, deren Volumen das Volumen der Ausgangsreaktionsmasse wesentlich übersteigt. Nach Abkühlung des Kolbens wird das Polymerharz mit Hilfe einer Spatel aus dem Kolben entfernt und in einem Mörser zu Pulver von heller Farbe zerkleinert, welches einen ziemlich hohen Grad an Hydrophobie aufweist. Als Ergebnis des Versuchs wurden ca. 232 g eines beschränkt in Wasser löslichen Polymers - Polytriethylenglykol-Guanidincarbonat mit einer charakteristischen Viskosität von [η] = 0,065 dl/g gewonnen (gemessen bei 25 °C, in einer 0,4 N wäßrigen NaCl-Lösung).
Elementaranalyse des gewonnene Polymers:

Berechnet für $C_8N_3O_5H_{17}$ : C - 40,85 %, N - 17,87 %, O - 34,04 %, H - 7,23 %
Gefunden: C - 41,31 %, N - 17,65 %, H - 7,03 %.

Die Analyse beweist, daß die Ergebnisse des Experimentes, wie gewünscht, mit der Berechnung übereinstimmen. Für das gewonnene Polymer - Polytriethylenglykol-Guanidinkarbonat - wurde der Wert der Mindesthemmkonzentration (µg/ml) für E-Coli-Bakterien bestimmt. Der Wert der MHK beträgt 20 mg/ml, was die biozide Aktivität des gewonnenen Polymers bestätigt.

Tabelle

| Eigenschaften synthetisierter Polyoxyalkylen-Guanidin-Hydrochloride | | | | |
|---|---|---|---|---|
| Muster Nr. | Charakteristische Viskosität [η] =dl/g gemessen in 0,1 n NaCl-Lösung bei 25 °C | Gewichtsmittel der relativen Molekül-masse Mw = | Bakterizidität Minimale Hemmkonzentration MHK in % (E. coli, Stamm 2590) | Toxizität Orale Dosis $LD_{50}$ mg/kg (bei Ratten) |
| Prototyp | 0,1 | 10000 | 0,0007 | 2500 |
| 1 a) | 0,04 | 2500 | 0,003 | |
| 1 b) | 0,07 | 5800 | 0,0015 | |
| 1 c) | 0,085 | 9100 | 0,001 | 3000 |
| 2 | 0,11 | 11800 | 0,0003 | 3100 |
| 3 a) | 0,045 | 3000 | 0,002 | |
| 3 b) | 0,12 | 12500 | 0,0001 | 3150 |
| 4 | 0,13 | 13500 | 0,00007 | 3250 |

## Patentansprüche

1. Biozide polymere Guanidinderivate auf der Basis von Diaminen, welche Oxyalkylenketten zwischen zwei Aminogruppen enthalten, **dadurch gekennzeichnet, daß** die Guanidinderivate ein Produkt der Polykondensation eines Guanidin-Säureadditionssalzes mit Diaminen, welche Polyoxyalkylenketten zwischen zwei Aminogruppen enthalten, darstellen.

2. Biozide Polymere nach Anspruch 1, **dadurch gekennzeichnet, daß** als Vertreter der Reihe der Polyoxyalkylen-Guanidin-Salze solche unter Einsatz von Triethylenglykoldiamin (relative Molekularmasse 148), von Polyoxypropylendiamin (relative Molekularmasse 230) sowie von Polyoxyethylendiamin (relative. Molekularmasse 600) sind

3. Verfahren zur Gewinnung von Polyoxyalkylen-Guanidinsalzen unter Verwendung von Diaminen, welche zwischen zwei Aminogruppen eine Polyoxyalkylenkette enthalten, in einem flüssigen Medium, **dadurch gekennzeichnet, daß** die flüssigen Diamine der Polykondensation mit Guanidin-Salzen im Medium von flüssigen Diamin unter ständiger Durchmischung von Beginn bis zum Abschluß der Reaktion bei erhöhter Temperatur und mit einer Dauer der Polykondensation von 9-16 Stunden, je nach Art des verwendeten Diamins, unterworfen werden.

4. Verfahren nach Anspruch 3, **dadurch gekennzeichnet, daß** die Polykondensationsreaktion bei einem Ausgangsmolverhältnis der Reagenzien von 1:1 durchgeführt wird.

5. Verfahren nach Anspruch 3 oder 4, **dadurch gekennzeichnet, daß** die Polykondensationsreaktion bei Temperaturen von 140 - 190 ° C durchgeführt wird.

6. Verfahren zur Gewinnung von Polyoxyalkylen-Guanidin-Hydrochloriden, **dadurch gekennzeichnet, daß** die Reaktion der Polykondensation von Guanidin-Hydrochlorid und flüssigen Diaminen mit einer Polyoxyalkylen-Gruppe zwischen den zwei Aminogruppen bei einem Ausgangsmolverhältnis der Reagenzien von 1 : 1 in einem flüssigen Reaktionsmedium (im Medium von flüssigem Diamin) unter ständiger Durchmischung von Beginn bis zum Abschluß der Reaktion bei Temperaturen von 150 - 170 °C und mit einer Dauer der Polykondensation von 18 — 25 Stunden je nach Art des verwendeten Diamins erfolgt.

7. Verfahren nach Anspruch 6, **dadurch gekennzeichnet, daß** eine Wechselwirkung von Guanidin-Hydrochlorid mit Triethylenglykoldiamin mit einer relativen Molekularmasse von 148 bei Temperaturen: von Beginn mit 150 °C 5 Stunden lang und anschließend von 170 °C 13 Stunden lang oder bei einer Temperatur von 150 °C innerhalb von 25 Stunden stattfindet.

8. Verfahren nach Anspruch 6, **dadurch gekennzeichnet, daß** eine Wechselwirkung von Guanidin-Hydrochlorid mit Polyoxypropylendiamin mit einer relativen Molekularmasse von 230 bei einer Temperatur von 150 °C innerhalb von 2 Stunden und anschließend mit einer Temperatur von 170 °C innerhalb von 18 Stunden stattfindet.

9. Verfahren nach Anspruch 6, **dadurch gekennzeichnet, daß** eine Wechselwirkung von Guanidin-Hydrochlorid mit Polyoxyethylendiamin mit einer relativen Molekularmasse von 600 bei einer Temperatur von 150 °C innerhalb von 25 Stunden stattfindet.

10. Biozide Polymere hergestellt nach einem Verfahren gemäß einem der Ansprüche 3 bis 9.

11. Biozide Polymere hergestellt nach dem Verfahren gemäß Anspruch 6.

## Claims

1. A biocidal polymer guanidine derivative based on diamines which include two amino groups and oxyalkyl chains therebetween, **characterized in that** the guanidine derivatives are a product of a polycondensation of a guanidine acid addition salt with diamines which include polyalkyl chains between two amino groups.

2. The biocidal polymer according to claim 1, **characterized in that** representatives of a number of polyoxyalkylene guanidine salts are such that have triethylene glycol diamine (relative molecular mass 148), polyoxy propylene diamine (relative molar mass 230), and polyoxyethylene diamine (relative molar mass 600).

3. A process for producing polyoxyalkylene guanidine salts using diamines which are included between two amino groups in a liquid medium, **characterized in that** the liquid diamines are exposed to the polycondensation with guanidine salts in a medium of liquid diamine under permanent thorough mixing from the beginning to the completion of the reaction at an increased temperature and at the duration of the polycondensation of 9 - 16 hours depending of the diamine used.

4. The process according to claim 3, **characterized in that** the polycondensation reaction is performed at an original mol relation of the reagents of 1:1.

5. The process according to claim 3 or 4, **characterized in that** the polycondensation reaction is performed at temperatures between 140 - 190 °C.

6. A process for producing polyoxyalkylene guanidine hydrochlorides, **characterized in that** the reaction of the polycondensation of guanidine hydrochloride and liquid diamines with the polyoxyalkylene group and the two amino groups occurs at an original mol relation of reagents of 1:1 in a liquid reaction medium (in the medium of liquid diamine) under permanent thorough mixing from the beginning to the completion of the reaction at temperatures of 150 - 170 °C and at the duration of the polycondensation of 18 - 25 hours depending on the diamines used.

7. The process according to claim 6, **characterized in that** the reciprocal effect of guanidine hydrochloride with triethylene glycol diamine having a relative molar mass of 148 occurs at temperatures: from the beginning at 150 °C for the duration of 5 hours and subsequently at 170 °C for the duration of 13 hours, or at a temperature of 150 °C within of 25 hours.

8. The process according to claim 6, **characterized in that** the reciprocal effect of guanidine hydrochloride with polyoxypropylene diamine having a relative molar mass of 230 occurs at a temperature of 150 °C within of 2 hours and, subsequently, at a temperature of 170 °C within of 18 hours.

9. The process according to claim 6, **characterized in that** the reciprocal effect of guanidine hydrochloride occurs with polyoxyethylene diamine having a relative molar mass of 600 at a temperature of 150 °C within of 25 hours.

**10.** Biocidal polymers produced according one of the claims 3-9.

**11.** Biocidal polymers produced according claim 6.

## Revendications

**1.** Dérivés polymères biocides de guanidine à partir de diamines, qui contiennent des chaînes oxyalcéniques entre deux groupes amino, **caractérisés en ce que** les dérivés de guanidine représentent un produit de la polycondensation d'un sel acide d'addition de la guanidine avec des diamines, qui contiennent des chaînes polyoxyalcéniques entre deux groupes amino.

**2.** Polymères biocides selon la revendication 1, **caractérisé en ce qu'**en tant que représentants de la série des sels de polyoxyalcényleguanidine figurent ceux intervenant avec l'emploi de triéthylèneglycoldiamine (masse moléculaire relative de 148), de polyoxypropylènediamine (masse moléculaire relative 230) ainsi que de polyoxyéthylènediamine (masse moléculaire relative de 600).

**3.** Procédé pour l'obtention de sels de polyoxyalcénylguanidine par application de diamines, qui contiennent une chaîne polyoxyalcénique entre deux groupes amino, dans un milieu liquide, **caractérisé en ce que** les diamines liquides sont soumises à la polycondensation de sels de guanidine dans le milieu liquide de la diamine liquide, avec mélange poussé permanent du début à la fin de la réaction à température élevée et avec une durée de polycondensation de 9 à 16 heures, selon le type de diamine utilisé.

**4.** Procédé selon la revendication 3, **caractérisé en ce que** la réaction de polycondensation est réalisée selon un rapport molaire initial des réagents de 1 :1.

**5.** Procédé selon la revendication 3 ou 4, **caractérisé en ce que** la réaction de polycondensation est réalisée à des températures de 140 à 190 °C.

**6.** Procédé pour l'obtention d'hydrochlorures de polyoxyalcénylguanidine, **caractérisé en ce que** la réaction de polycondensation d'hydrochlorure de guanidine et de diamines liquides avec un groupe polyoxyalcényle entre les deux groupes amino intervient selon un rapport molaire initial des réagents de 1 : 1 dans un milieu réactionnel liquide (milieu de l'amine liquide) avec mélange poussé permanent du début à la fin de la réaction à des températures de 150-170 °C et avec une durée de polycondensation de 18 à 25 heures, selon le type de diamine utilisé .

**7.** Procédé selon la revendication 6, **caractérisé en ce qu'**une interaction entre l'hydrochlorure de guanidine et la triéthylèneglycolamine présentant une masse moléculaire relative de 148 intervient aux températures suivantes : au début à 150 °C pendant 5 heures, puis à 170 °C pendant 13 heures ou à une température de 150 °C pendant une période de 25 heures.

**8.** Procédé selon la revendication 6, **caractérisé en ce qu'**une interaction entre l'hydrochlorure de guanidine et la polyoxypropylènediamine présentant une masse moléculaire relative de 230 intervient à une température de 150 °C pendant 2 heures, puis à une température de 170 °C pendant 18 heures.

**9.** Procédé selon la revendication 6, **caractérisé en ce qu'**une interaction entre l'hydrochlorure de guanidine et la polyoxyéthylènediamine présentant une masse moléculaire relative de 600 intervient à une température de 150 °C pendant une période de 25 heures.

**10.** Polymères biocides fabriqués par un procédé selon l'une des revendications 3 à 9.

**11.** Polymères biocides fabriqués par le procédé selon la revendication 6.